# EUROPEAN PATENT SPECIFICATION

(11) **EP 3 052 149 B1**
(45) Date of publication and mention of the grant of the patent: **06.11.2019**
(21) Application number: 14848585.7
(22) Date of filing: 26.09.2014
(51) Int. Cl.: C08L 29/04, C08K 13/02, C08K 3/10, C08J 3/20, C08K 5/00, C08J 5/18, A61F 13/84, C08L 3/02, C08L 67/00, C08L 71/02

(54) **PERSONAL ABSORBENT ARTICLE WITH ACTIVE AGENT**
WIRKSTOFF-GELADENER ABSORBIERENDER ARTIKEL FÜR DEN PERSÖNLICHEN GEBRAUCH
ARTICLE ABSORBANT D'USAGE PERSONNEL COMPRENANT UN AGENT ACTIF

(30) Priority: 30.09.2013 US 201361884574 P
(43) Date of publication of application: 10.08.2016
(73) Proprietor: Kimberly-Clark Worldwide, Inc., Neenah, Wisconsin 54956 (US)
(72) Inventor: ZHUANG, Shiming, Neenah, Wisconsin 59456 (US); VONGSA, Rebecca, Ann, Neenah, Wisconsin 54956 (US); ZHOU, Peiguang, Neenah, Wisconsin 54956 (US); LONG, Andrew, Mark, Neenah, Wisconsin 54956 (US); KOENIG, David, William, Neenah, Wisconsin 54956 (US); TYRRELL, David, John, Neenah, Wisconsin 54956 (US)
(74) Representative: Dehns
(86) International application number: PCT/US2014/057798
(87) International publication number: WO 2015/048505

(56) References cited:
- WO-A2-2009/126512
- WO-A2-2010/001269
- US-A1- 2001 006 677
- US-A1- 2007 281 003
- US-A1- 2008 142 023
- US-A1- 2012 021 026
- US-A1- 2012 201 865
- US-B1- 6 375 963
- US-B1- 6 375 963

## Description

### BACKGROUND

Active agents, such as antimicrobial agents, have been used in conjunction with many products. In particular, since viral outbreaks such as SARS, bird flu and norovirus have been widely publicized, consumers have an increased interest in a wide array of products having an antimicrobial agent applied thereto; products such as wipes, shoe inserts, athletic clothing, personal care products, hospital equipment, sports equipment, etc. Products such as absorbent articles meant for bodily fluids (e.g. disposable diapers, pads and incontinence garments) may offer an odor-control benefit when an antimicrobial agent is applied thereto.

Currently, the application of an antimicrobial agent onto a select portion of an absorbent personal-care article is achieved by applying it as a lotion, cream, or spray formulation. However, due to the application method, and/or the incompatibility and/or poor solubility of the antimicrobial agent(s) in the formulation, getting effective concentrations of the active agents onto the personal care product is challenging.

There remains a need for a better way to apply an active agent such as an antimicrobial agent to an absorbent personal-care product. For instance, there is a desire for an application method that does not require several additional steps in the manufacture of such a product (e.g. spraying, slot-coating, or the like). There is also a desire for a method of applying an active agent to such a product so that it demonstrates higher efficacy than is currently available.

### SUMMARY

The present disclosure relates to a personal absorbent article comprising an absorbent member disposed between a water-impermeable backsheet and a water-permeable liner. An extruded water-soluble article described in the first aspect of the disclosure, in the form of a film and comprising a homogeneous material comprising a water-soluble polyvinyl alcohol (PVOH) having an extrusion temperature of 50 to 150°C and between 0.1% to 50% by weight of an antimicrobial active agent attached to the liner or to a surface of the absorbent member adjacent the liner.

Other features and aspects of the invention will become apparent by consideration of the detailed description and accompanying drawings.

### BRIEF DESCRIPTION OF THE DRAWINGS

The foregoing and other features and aspects of the present invention and the manner of attaining them will become more apparent, and the invention itself will be better understood by reference to the following description, appended claims and accompanying drawings, where;
FIG. 1 is a chart showing the dissolution time for one embodiment of a film according to the present disclosure;
FIGS. 2-4 are charts showing zone of inhibition on films containing various biocides of the present disclosure;
FIG. 5 is a chart showing the stress and strain properties of films having varying percentages of a first embodiment of an antimicrobial agent;
FIG. 6 is a chart showing the modulus and toughness of the films of FIG. 5;
FIG. 7 is a chart showing the stress and strain properties of films having varying percentages of a second embodiment of an antimicrobial agent;
FIG. 8 is a chart showing the modulus and toughness of the films of FIG. 7;
FIG. 9 is a chart showing the stress and strain properties of films having varying percentages of a third embodiment of an antimicrobial agent;
FIG. 10 is a chart showing the modulus and toughness of the films of FIG. 9;
FIG. 11 is a side elevation of one embodiment of a laminate according to the present disclosure;
FIG. 12 is an exploded side elevation of one embodiment of a personal absorbent article;
FIG. 13 is a side cross-sectional view of one embodiment of an absorbent article according to the present disclosure;
FIG. 14 is a schematic showing various steps of a zone of inhibition test according to the disclosure; and
FIG. 15 is a schematic showing how test material is spread onto a medium in the test of FIG. 14.

### DETAILED DESCRIPTION

The term "laminate" refers to a material where a film structure is adhesively or non-adhesively bonded to a web such as a nonwoven or tissue material.

The term "meltblown fibers" refers to fibers formed by extruding a molten thermoplastic material through a plurality of fine, usually circular, die capillaries as molten threads or filaments into a high velocity, usually heated, gas (e.g., air) stream which attenuates the filaments of molten thermoplastic material to reduce their diameter. In the particular case of a coform process, the meltblown fiber stream intersects with one or more material streams that are introduced from a different direction. Thereafter, the meltblown fibers and other optional materials are carried by the high velocity gas stream and are deposited on a collecting surface. The distribution and orientation of the meltblown fibers within the formed web is dependent on the geometry and process conditions. Exemplary meltblown processes are described in various patents and publications, including NRL Report 4364, "Manufacture of Super-Fine Organic Fibers" by V. A. Wendt, E. L. Boone and C. D. Fluharty; NRL Report 5265, "An Improved Device For the Formation of Super-Fine Thermoplastic Fibers" by K. D. Lawrence, R. T. Lukas and J. A. Young; and U.S. Patent No. 3,849,241 to Butin et al. and U.S. Patent No. 5,350,624 to Georger et al..

The terms "nonwoven" and "nonwoven web" refer to materials and webs of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblown processes, spunbond processes, air laying processes, wet layering processes and bonded-carded-web processes.

The term "personal care absorbent articles" or "absorbent articles" in the context of this disclosure includes diapers, diaper pants, training pants, absorbent underpants, incontinence products, and urinary shields.

The terms "spunbond" and "spunbond fiber" refer to fibers which are formed by extruding filaments of molten thermoplastic material from a plurality of fine, usually circular, capillaries of a spinneret, and then rapidly reducing the diameter of the extruded filaments.

The term "% by weight," "weight %," "wt%" or derivative thereof, when used herein, is to be interpreted as based on the dry weight, unless otherwise specified.

These terms may be defined with additional language in the remaining portions of the specification.

The present disclosure is generally directed to an extruded, water-soluble, thermoplastic article into which an active agent has been incorporated. The thermoplastic water-soluble, polymer from which the article is made has an extrusion temperature of 90°C to 150°C. The combination of the polymer and active agent(s) is a homogeneous blend having an extrusion temperature of 50°C to 150°C, more preferably up to 125°C. The articles made from the homogeneous blend include films, fibers, pellets, or other extruded shapes.

### Materials

The materials from which the water-soluble, thermoplastic material of the present disclosure is made generally include a polymer and one or more active agents. Other optional materials that improve the performance, look, feel and/or durability may be added to the thermoplastic material.

**Polymer:** The polymer used in the present disclosure is polyvinyl alcohol (PVOH). Suitable polymers have an extrusion temperature of 90°C to 150°C.

One desirable polymer is a highly amorphous vinyl alcohol polymer, sold as "NICHIGO G-POLYMER," available from Soarus L.L.C., Arlington Heights, Illinois. This particular polymer has a molecular weight of 10,000 to 50,000, and a relatively low crystallinity of 5 to 25%.

In one aspect, a copolymer such as ethylene vinyl acetate (EVA) may be combined with the base polymer. It is contemplated that the article of the present disclosure may include up to 30% by weight EVA. EVA aids in extrusion process, provides a means to control the water dissolution speed, and lowers the overall cost of the extruded material.

**Active Agent:** The active agent is made from one or more antimicrobial agents. Suitable antimicrobials include biocides such as benzalkonium chloride ("BAC"), didecyl dimethyl ammonium chloride ("DDAC"), and zeolite ("CWT-A"). Other possible active agents include: isothiazolone, alkyl dimethyl ammonium chloride, a triazine, 2-thiocyanomethylthio benzothiazol, methylene bis thiocyanate, acrolein, dodecylguanidine hydrochloride, a chlorophenol, a quaternary ammonium salt, glutaraldehyde, a dithiocarbamate, 2-mercatobenzothiazole, para-chloro-meta-xylenol, silver, chlorohexidine, polyhexamethylene biguanide, a N-halamine, triclosan, a phospholipid, an alpha hydroxyl acid, 2,2-dibromo-3-nitrilopropionamide, 2-bromo-2-nitro-1,3-propanediol, farnesol, iodine, bromine, hydrogen peroxide, chlorine dioxide, a botanical oil, a botanical extract, benzalkonium chloride, chlorine, sodium hypochlorite, or combinations thereof.

The amount of active agent that is loaded into an article is limited due to the integrity of the resulting article structure. If there is too much active agent in an article, it may be unduly weakened. The sum of the active agent(s) is present in a total amount of 0.1% to 50% by weight of the article, or a total amount of 1% to 20% by weight of the article. In another aspect, the sum of the active agent(s) is present in a total amount of 2% to 10% by weight of the article.

**Optional Materials:** Besides the components noted above, still other additives may also be incorporated into the composition, such as fragrances, melt stabilizers, dispersion aids (e.g., surfactants), processing stabilizers, heat stabilizers, light stabilizers, UV stabilizers, antioxidants, heat aging stabilizers, whitening agents, antiblocking agents, antistatic agents, bonding agents, lubricants and colorants.

In one aspect of the present disclosure, the extruded water-soluble article includes up to 50% thermoplastic starch by weight. The starch acts as a filler to reduce the overall cost of the extruded article. The extruded article may contain as much as 30% starch. When a starch is employed, the amount of such additional material may range from 0.1 wt. % to 50 wt. % of the homogeneous blend, in some embodiments from 0.5 wt. % to 40 wt. %, and in some embodiments, from 1 wt. % to 30 wt. %.

Dispersion aids may also be employed to help create a uniform dispersion of the active agent/plasticizer. When employed, the dispersion aid(s) typically constitute from 0.01 wt. % to 10 wt. % of the homogeneous blend, in some embodiments from 0.1 wt. % to 5 wt. %, and in some embodiments, from 0.5 wt. % to 4 wt. %.

The composition may also contain a preservative or preservative system to inhibit the growth of microorganisms over an extended period of time. Suitable preservatives may include, for instance, alkanols, disodium EDTA (ethylenediamine tetraacetate), EDTA salts, EDTA fatty acid conjugates, isothiazolinone, benzoic esters (parabens) (e.g., methylparaben, propylparaben, butylparaben, ethylparaben, isopropylparaben, isobutylparaben, benzylparaben, sodium methylparaben, and sodium propylparaben), benzoic acid, propylene glycols, sorbates, urea derivatives (e.g., diazolindinyl urea), and so forth. Other suitable preservatives include those sold by Sutton Labs, such as "Germall 115" (amidazoiidinyl urea), "Germall II" (diazolidinyl urea), and "Germall Plus" (diazolidinyl urea and iodopropynyl butylcarbonate). Another suitable preservative is Kathon CG.RTM., which is a mixture of methylchloroisothiazolinone and methylisothiazoiinone available from Rohm & Haas; Mackstat H 66 (available from McIntyre Group, Chicago, III.). Still another suitable preservative system is a combination of 56% propylene glycol, 30% diazolidinyl urea, 11 % methylparaben, and 3% propylparaben available under the name GERMABEN.RTM. II from International Specialty Products of Wayne, N.J.

To better enhance the benefits to consumers, other optional ingredients may also be used. For instance, some classes of ingredients that may be used include, but are not limited to: antioxidants (for product integrity); astringents-cosmetic (for inducing a tingling sensation on skin); colorants (for imparting color to the product); deodorants (for reducing or eliminate unpleasant odor and protect against the formation of malodor on body surfaces); fragrances (for consumer appeal); skin conditioning agents; and skin protectants (a drug product which protects injured or exposed skin or mucous membrane surface from harmful or annoying stimuli).

### Method of Manufacture

A method of making an extruded article may include the following steps. First, a homogenous blend is formed by combining the polymer with at least one active agent and possibly, one or more of the optional ingredients described herein. In one desired embodiment, the polymer is an amorphous, water-soluble vinyl alcohol as described herein. Second, the homogeneous blend is extruded to form an article.

The homogeneous blend has an extrusion temperature of 50°C to 150°C, or possibly, 90°C to 125°C. This low extrusion-temperature profile is desirable because some active agents of interest have poor thermal stability. By using a low extrusion-temperature, a wider variety of active agents may be incorporated into the homogenous blend.

Exemplary manufacturing equipment, a method of making articles, and exemplary articles are described herein.

**Extrusion Method:** The composition of the present disclosure is formed by processing the components together in a melt-blending device (e.g., extruder). The mechanical shear and heat provided by the device allows the components to be blended together in a highly efficient manner without the use of a solvent. Batch and/or continuous melt blending techniques may be employed in the present disclosure. For example, a mixer/kneader, Banbury mixer, Farrel continuous mixer, single-screw extruder, twin-screw extruder, roll mill, etc., may be utilized. One particularly suitable melt-blending device is a twin-screw extruder (e.g., PRISM USALAB x16, available from Thermo Electric Co., Inc., New Jersey).

The polymer and the active agent(s), along with any optional ingredients, form a homogeneous blend. For example, the materials may be blended at a shear/pressure and temperature sufficient to ensure adequate mixing (e.g., at or above the softening point of the polymer), but without adversely impacting the physical properties of the active agent. For example, melt-blending typically occurs at a temperature of from 50°C to 150°C., in some embodiments, from 90°C to 130°C, and in some embodiments from 110°C to 125°C. These lower processing temperatures prevent degradation of the active agent.

Once formed, the homogeneous blend may be used to create a variety of forms, such as films, fibers, rods, bars or other shapes.

Films: In one particular embodiment, the homogeneous blend is formed into a film, either alone or in conjunction with an additional film-forming material. The film may be used in a wide variety of applications, such as a carrier of active agents for medical products, garments, absorbent articles, etc. The film may have a mono- or multi-layer configuration. Any known extrusion technique may be used to form a film from the compounded material such as extrusion coating, coextrusion of the layers, or any conventional layering process.

The process to make the antimicrobial reservoir film is relatively fast considering the high amounts of active agent that can be added to the extrusion process. In one particular embodiment, the film may be formed by flat die extrusion technique. Processes for producing such extrusions are described, for instance, in U.S. Pat. No. 7,666,337 to Yang et al.; U.S. Pat. No. 5,091,228 to Fuji et al; and U.S. Pat. No. 4,136,145 to Fuchs et al..

Regardless of how the film is formed, it may be optionally oriented in one or more directions to further improve film uniformity and reduce thickness. For example, the film may be immediately reheated to a temperature below the melting point of one or more polymers in the film, but high enough to enable the composition to be drawn or stretched. In the case of sequential orientation, the "softened" film is drawn by rolls rotating at different speeds or rates of rotation such that the sheet is stretched to the desired draw ratio in the longitudinal direction (machine direction). The film may be made into thicknesses ranging from 0.01 mm up to 1 mm, or in other aspects, from 0.05 mm to 0.20 mm.

The multi-layer film may contain from two (2) to nine (9) layers, and in some embodiments, from three (3) to five (5) layers. In one example, the multi-layer film has one base layer and one skin layer. The base layer and/or skin layer may contain the active agent(s). The ratio between the layers may range from 1 to 20.

In another example, there is a three-layered film having a core layer "C" that is contains an active agent as described herein. The outer skin layers "S" may act as a protective layer to the core. The ratio between the layers may range from 2% to 98% of the core layer and from 10% to 90% of the two combined skin layers. For instance, the core layer may be up to 30%, up to 40%, up to 50%, up to 60%, or up to 70% of the total thickness of the multi-layer film. Each skin layer may be up to 15%, or up to 25%, or up to 35% of the total thickness of the multi-layer film.

The film, either mono- or multi-layered, may be wound and stored on a take-up roll. Various additional potential processing and/or finishing steps known in the art, such as slitting, treating, aperturing, printing graphics may be performed.

In one aspect, the extruded water-soluble film has a basis weight of 5 g/m² to 500 g/m². In another aspect, the water-soluble film has a basis weight of 20 g/m² to 200 g/m².

In one aspect, the extruded water-soluble film has a tensile strength of 0.5 MPa to 50 MPa according to the Tensile Test of the present disclosure. In another aspect, the film has a tensile strength of 1 MPa to 25 MPa according to the same test.

In one aspect, the extruded water-soluble film has a water dissolution speed from 5 seconds to 30 minutes as determined by the Water Dissolution Test of the present disclosure. In another aspect, the extruded water-soluble article film has a water dissolution speed of 30 seconds to 5 minutes as determined by the same test.

In one aspect, the extruded water-soluble film has an elongation of 5% to 500% according to the Tensile Test of the present disclosure. In another aspect, the film has an elongation of 10% to 100% according to the same test.

Articles: The homogeneous blend of the present invention may also be used to form other types of articles. In one aspect, the extruded water-soluble article is a rod having a circular- or elliptical-shaped extrusion profile. In another aspect, the extruded water-soluble article is a rod having the geometric extrusion profile of a polygon with three to ten sides (e.g. a triangle to a decagon). The rod may be cut into pellets for later processing.

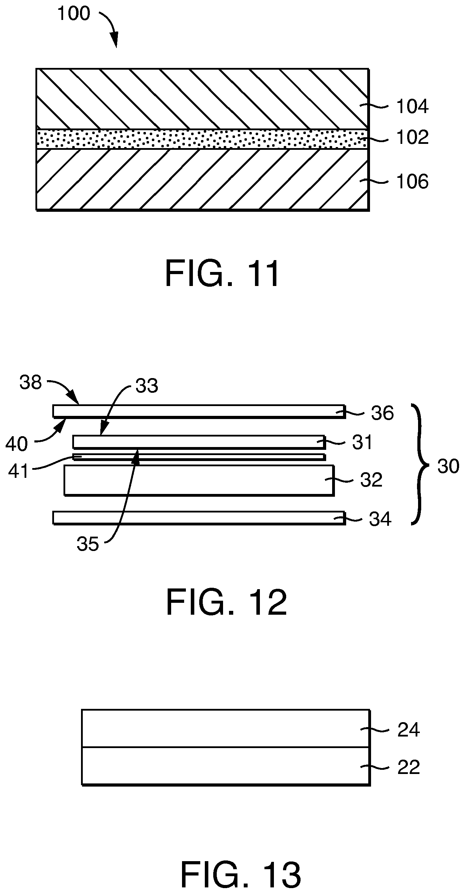

Referring to **Fig. 13****,** a laminate may be formed by extruding the homogeneous blend 24 onto a carrier substrate 22, forming a bond therebetween. The carrier substrate 22 may be a nonwoven or woven material.

### Applications

**Absorbent Articles:** The film described above is used in an absorbent article of the present invention. An "absorbent article" generally refers to any article capable of absorbing water or other fluids. The absorbent articles are personal care absorbent articles selected from diapers, diaper pants, training pants, absorbent underpants, incontinence products, and urinary shields. Several examples of such absorbent articles are described in U.S. Patent Nos. 5,649,916 to DiPalma, et al.; 6,110,158 to Kielpikowski; 6,663,611 to Blaney, et al.. Still other suitable articles are described in U.S. Patent Application Publication No. 2004/0060112 A1 to Fell et al., as well as U.S. Patent Nos. 4,886,512 to Damico et al.; 5,558,659 to Sherrod et al.; 6,888,044 to Fell et al.; and 6,511,465 to Freiburger et al.. Materials and processes suitable for forming such absorbent articles are well known to those skilled in the art.

The present invention may be better understood with reference to the examples presented herein.

**First Exemplary Absorbent Article:** Referring to **FIG. 12****,** in one aspect of the disclosure, a personal absorbent article 30 includes an absorbent member 32 sandwiched between a water-impermeable backsheet 34 and a water-permeable liner 36, wherein liner 36 has a body-facing surface 38 and an opposite outward-facing surface 40. A film 41 of the present disclosure is attached to either the outward-facing surface 40 of the liner or a surface 42 of the absorbent member 32 that is adjacent liner 36. Desirably, film 41 is in direct contact with liner 36. Should a multi-layer film be used for film 41, the layer containing the largest amount of active agent is adjacent liner 36 so that the active agent can more easily leach through the liner to contact the wearer's body.

As described, film 41 is made from materials that include a water-soluble, polymer that may have an extrusion temperature of 90 to 150°C; a plasticizer; and one or more volatile active agents in a total amount of 0.1 % to 50% by weight of the article.

**Second Exemplary Absorbent Article:** Referring to **FIG. 11****,** in one aspect, the film of the present disclosure is laminated to other layers (e.g., nonwoven or cellouse fiber based web materials). One particular application of a laminate structure is that of a three-layer wipe 100. In this embodiment, the core layer 102 is a film containing at least the active agent(s) of the present disclosure. Desirably, the outer layers 104 and 106 that surround the core layer are natural or synthetic fiber based web materials (e.g. tissue, paper, spunbond, spunbond-meltblown-spunbond composite, coform, airlaid, etc.).

### Experimental Data

Provided is experimental data for three antimicrobial agents that act as biocides, namely, zeolite, benzalkonium chloride, and didecyl dimethyl ammonium chloride. Tests were performed on the various codes for each biocide to determine dissolution, zone of inhibition, and mechanical properties.

**TABLE 1**

| **Category** | **Code** | **Additives** | |
|---|---|---|---|
| | | **%** | |
| **Antimicrobial Agents** | GP25-C00 | 0 | Inorganic biocide (zeolite) Sourced from: Jishim Tech Co., Lid (Korea), CWT-A (brand name). |
| | GP25-C05 | 5 | |
| | GP25-C10 | 10 | |
| | GP25-C20 | 20 | |
| | GP25-C50 | 50 | |
| | GP25-B01 | 1 | BAC (benzoalkonium chloride) Sourced from Mason Chemical, Company, IL, under NOBAC (brand name). |
| | GP25-B02 | 2 | |
| | GP25-B05 | 5 | |
| | GP25-B10 | 10 | |
| | GP25-D01 | 1 | DDAC (didecyl dimethyl ammonium chloride) Sourced from, Lonza Inc, NJ, BARDAC 2250 (brand name). |
| | GP25-D02 | 2 | |
| | GP25-D05 | 5 | |
| | GP25-D10 | 10 | |

Films containing the various amounts of the antimicrobial agents of Table 1 were made as follows. A twin-screw extruder (PRISM USALAB x16, available from Thermo Electric Co., Inc.) was used to make co-extruded film samples that contain an antimicrobial agent. The extruder specifications were as follows:
- 16 mm diameter screw
- L/D=40 (L=640 mm)
- 10 heating zones + die
- Maximum velocity = 1000 rpm
- Maximum pressure = 100 bar
- Maximum torque = 24 mN

The following extruder set-up was used to manufacture the experimental film:
- Flat slit die width: 152.40 mm (6")
- Flat slit die height (controls film thickness): 0.127 mm (0.010")

Each coextruded film included one of the active ingredients of **Table 1.**

The extruder feed zone was heated to 110°C, the following extruder zones 2-9 were heated to 125°C, and the die was heated to 130°C. The material was extruded.

### Dissolution Test:

### I. Preparation of specimens:

a. Cut 9 film specimens (approximately 0.75" x 2.5" i.e. 1.905 cm x 6.350 cm, or 0.07-0.12 g each). Record the mass of each specimen.
b. Match each specimen with a tall 2 oz (59.15 ml) glass jar and lid. Fill each jar with enough buffered water so that the water is 100 times the weight of the film. Three jars are to be filled with a pH 5 buffered solution, three with a pH 7, and 3 with a pH 9 buffered solution.
   i. The buffered solutions contain:
      1. pH 5: 990 g tap water, 10 g sodium citrate, 1.89 g citric acid
      2. pH 7: 990 g tap water, 10 g sodium citrate, 0.18 g citric acid
      3. pH 9: 990 g tap water, 10 g sodium citrate, 1.02 g triethanolamine
c. Heat one jar from each pH to 60°C, and one jar from each pH to 40°C. The last jar of each pH remains at room temperature (approx. 20°C)

### II. Testing of specimens:

a. Gather the film specimens, a stopwatch, a glass stir-rod, and the jars of buffered water.
b. Drop a film specimen into each jar, using the glass rod to submerge the film specimen if necessary. Do not drop the sample onto the wall of the jar, as the film will adhere and take longer to dissolve.
c. Start the timer immediately after submerging the film specimen.
d. Record the time that the film is 95%+ dissolved. Swirl the jar if necessary to check to see if the film is dissolved. Some films cloud the water and make it difficult to discern when the specimen is dissolved.

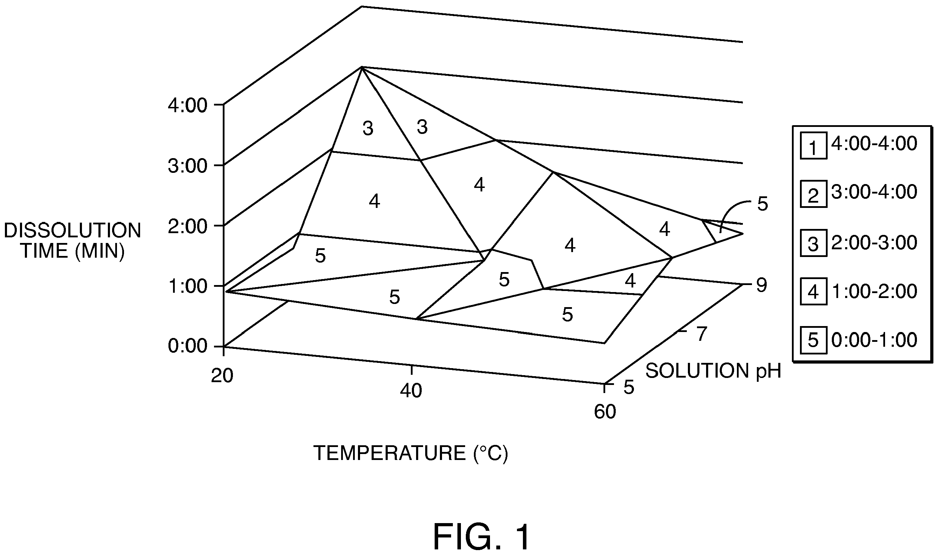

FIG. 1 is a three-dimensional graph showing the dissolution time for antimicrobial GP25-C05, at varying pH and temperature. The longest dissolution time of three minutes is shown at a condition of pH 9 and 20°C. In contrast, the shortest dissolution time of less than 1 minute is shown at a condition of pH 5 and 60°C.

**Zone of Inhibition Test:** In this test method, the test material is brought into contact with a known population of microorganisms on an agar plate for a specified period of time. At the end of the contact time, the area of inhibited colony formation around the test material is measured. The size of this area of no growth is a measure of leaching of the antimicrobial agent from the test material.

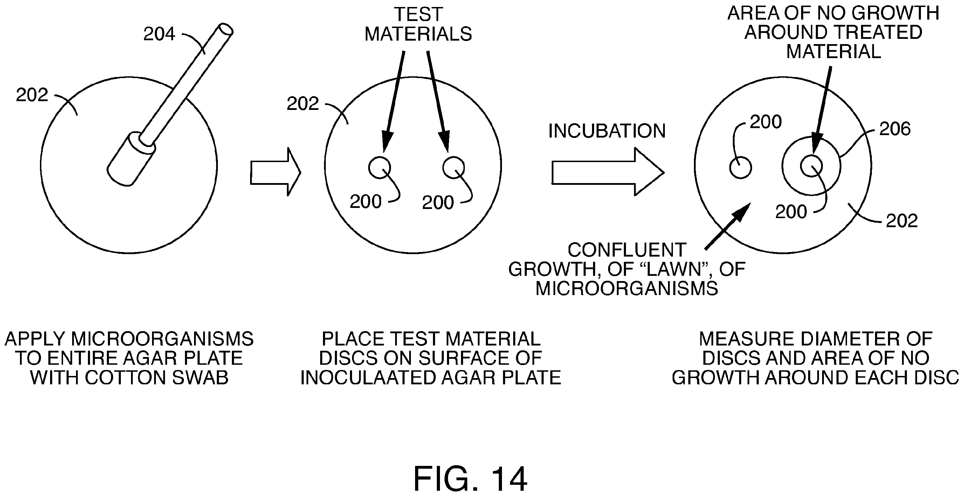

Referring to **FIG. 14****,** the test material 200 is cut into small discs and placed on an agar plate 202 evenly spread with a test microorganism with a cotton swab 204. The plates are incubated for 24 hours at ideal growth conditions. Following incubation, the diameter of the circle of no growth 206 around the disc 200 is measured. The zone of inhibition is reported as the difference between the sample disc diameter and the average of the measured no growth zone diameters.

### Materials and Reagents:

- Microorganisms: frozen stock of Staphylococcus aureus (ATCC 27660) and Pseudomonas aeruginosa (ATCC 15442).
- Mueller-Hinton agar (MHA) plates or equivalent plated media. Prepare following manufacturer's directions. Store at 4± 2 °C. Alternatively, pre-made plates can be utilized.
- Mueller-Hinton broth (MHB) or equivalent liquid media. Prepare following manufacturer's directions. Store at 4 ± 2 °C. Alternately, pre-made media can be utilized.

- Sterile cotton swabs or equivalent.
- Sterile forceps.
- Positive control disc: Vanocymicin susceptibility discs (6 mm), 30 µg/disc (BD and Company; Sparks, Maryland).
- Test material, cut into 8 mm discs.
- Calipers or other measuring device.
- Other ancillary lab supplies.

### Supply Set-up:

1. Label growth media plates appropriately for testing codes.
2. Sterilize test material discs with UV exposure in Laminar flow hood for 15 minutes (both sides of disc), if required.

### Inoculum:

1. Take appropriate measures to ensure culture purity.
2. *Staphylococcus aureus* or *Pseudomonas aeruginosa* is inoculated from an overnight plate or MHB into 5 ml of sterile MHB in a 35 °C incubator for 18 - 24 hrs.
3. The overnight culture is then adjusted using MHB to the 0.5 McFarland barium sulphate standards (1 x 108 CFU/ml) or approximately 0.15 OD with a 0.2 cm light path at 660 nm.
4. Discard the cell suspension if it is not used within 30 to 60 min after preparation.

### Zone of Inhibition Bioassay Procedure:

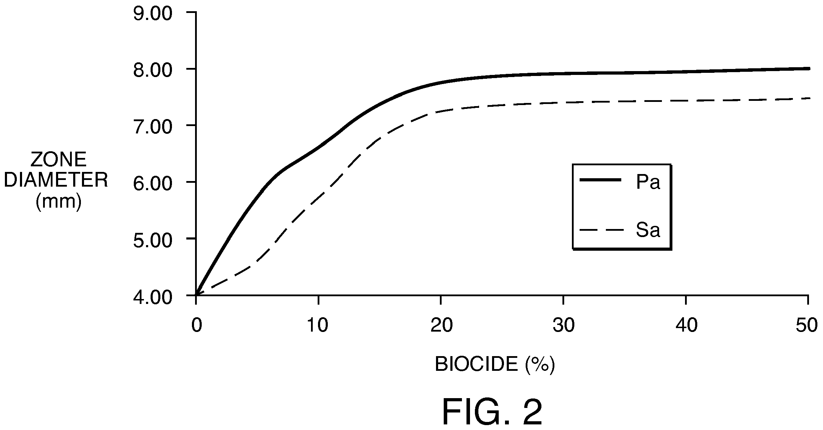

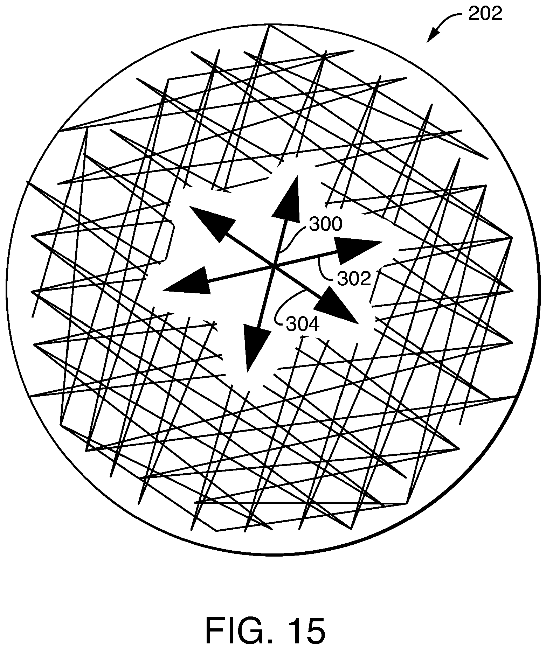

1. Pre-warm the MHA plates to room temperature. The number of plates required per strain will depend on the number of test materials to be tested and their anticipated zone inhibition diameters; discs should be placed on plates so that zones of inhibition do not overlap.
2. The surface of the plates should be dry. If not, dry the plates (with lids ajar) in a 35 °C incubator for 20 - 30 min just prior to inoculation. There should be no visible droplets of moisture on the surface of the agar or on the lids of the plates when they are inoculated.
3. Moisten a sterile applicator swab in the standardized cell suspension and express any excess moisture by rotating the swab against the glass above the liquid in the tube. Referring to FIG. 15, inoculate the entire surface of each agar plate 202, inoculating the surface completely in three different directions 300, 302, 304 to ensure uniform growth.
   (It is recommended that cotton swabs with wooden handles be used for this procedure. Swabs made of synthetic materials do not soak up sufficient suspension to inoculate the entire surface of the plate. Swabs with plastic handles bend when excess suspension is being expressed and may splatter liquid out of the tube.)
4. Repeat step 10.3 to inoculate additional plates as needed.
5. Store the inoculated plates at room temperature for 10 - 15 min to allow the medium to absorb the moisture from the inoculum.
6. Apply discs of test material to the surface of the inoculated medium with a sterile forceps and tap them to ensure that they are in complete contact with the agar surface. A positive control (vancomycin disc) and negative control (uncoated disc) should be used on each plate. All discs should be approximately the same distance from the edge of the plate and from each other (Figure 2). In addition, all the discs should be positioned so the area of no growth that may develop around them to do not overlap.
7. Invert the inoculated plates and incubate them at 35 °C for 18 - 24 hours.
8. Examine the plates from the back, viewed against a black background and illuminated with reflected light. With calipers, measure the diameter of each zone of inhibition to the nearest whole millimeter.

Calculation: The zone of inhibition is equal to the diameter of the no-growth area minus the diameter of the disc.

The inhibition zone sizes given in this test protocol are derived from test methods used at the Center for Disease Control as well as AATCC Method 147-1998 (19) based on the National Committee for Clinical Laboratory Standards (20-21) and ASTM E2149-01 step 12.2 (22). The diameters of zones of inhibition may vary depending on many factors including medium base, humidity, and the age of the medium. Thus, it is important to follow one protocol as closely as possible to obtain results comparable between labs, personnel, and experiments. It may be necessary to determine zone interpretative sizes for disc diffusion results that are appropriate to local conditions. These criteria may be determined with use of reference strains and known challenge compounds and amounts.

Results: **FIG 2****.** shows the result of the zone of inhibition testing for films containing antimicrobial agent CWT-A. This agent was more effective against *Pseudomonas aeruginosa* (Pa) than *Staphylococcus aureus* (Sa), but the effectiveness against both microbes plateaued when the film contained 20% or more of the antimicrobial agent.

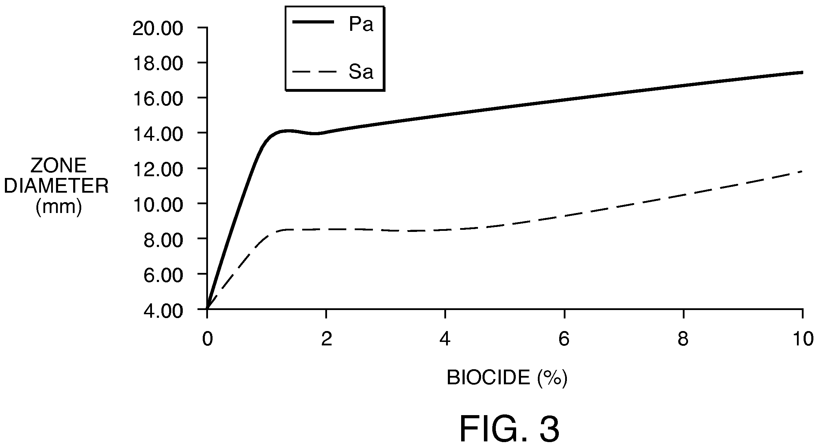

**FIG. 3** shows the result of the zone of inhibition testing for films containing antimicrobial agent DDAC. This agent was significantly more effective against *Staphylococcus aureus* (Sa) than *Pseudomonas aeruginosa* (Pa). The effectiveness against Sa microbes went from a zone of 4 mm to 14 mm between 0% and about 1% DDAC. When the film contained from about 1% and 10% DDAC, the zone of inhibition of the Sa microbes went from about 14 mm to about 17 mm. The effectiveness against Pa microbes went from a zone of 4 mm to about 7 mm between 0% and about 1% DDAC. When the film contained from about 1% and 10% DDAC, the zone of inhibition of the Pa microbes went from about 9 mm to about 12 mm, plateauing at about 9 mm between about 1% and 4% DDAC.

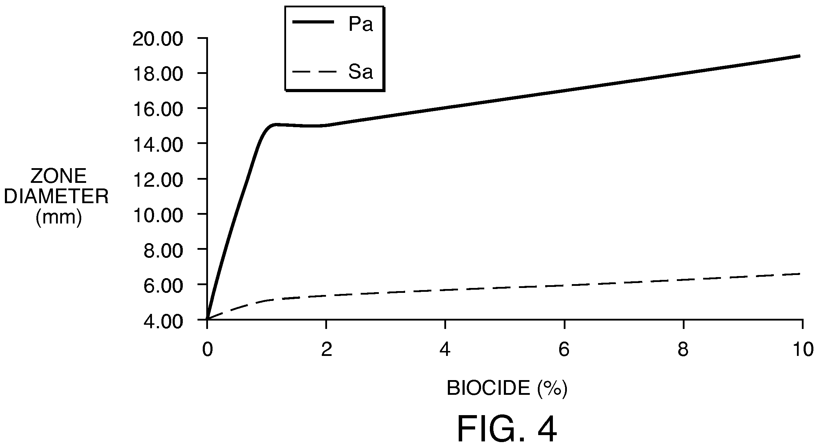

**FIG. 4** shows the result of the zone of inhibition testing for films containing antimicrobial agent BAC. This agent was much more effective against *Staphylococcus aureus* (Sa) than *Pseudomonas aeruginosa* (Pa). The effectiveness against Sa microbes went from a zone of 4 mm to 14 mm between 0% and about 1% BAC. When the film contained from about 1% and 10% BAC, the zone of inhibition of the Sa microbes went from about 15 mm to about 19 mm. The effectiveness against the Pa microbes went from a zone of 4 mm to about 6mm between 0% and about 10% BAC.

**Tensile Test:** Prior to testing, samples were initially conditioned at 75°F (24°C)/50% relative humidity for 24 hours. Thereafter, the strip tensile strength values were determined in accordance with ASTM Standard D-5034. A constant-rate-of-extension type of tensile tester was employed. The tensile testing system was a Synergie 200 tensile frame. The tensile tester was equipped with TESTWORKS 4.08B software from MTS Systems Corp. to support the testing. An appropriate load cell was selected so that the tested value fell within the range of 10-90% of the full scale load. The film samples were initially cut into dog-bone shapes with a center width of 3.0 mm before testing. The samples were held between grips having a front and back face measuring 25.4 millimeters x 76 millimeters. The grip faces were rubberized, and the longer dimension of the grip was perpendicular to the direction of pull. The grip pressure was pneumatically maintained at a pressure of 40 pounds per square inch (275.8 kPa). The tensile test was run using a gauge length of 18.0 millimeters and a break sensitivity of 40%. Five samples were tested by applying the test load along the machine-direction and five samples were tested by applying the test load along the cross direction. During the test, samples were stretched at a crosshead speed of about 127 millimeters per minute until breakage occurred. The modulus of elasticity, peak load, peak stress, elongation (percent strain at break), and energy per volume at break (total area under the stress-strain curve) were measured.

The tensile test results showed that the films have excellent mechanical properties for high-speed converting processes. This allows the films to easily be placed into articles such as the absorbent articles and laminates described herein.

### Test Results:

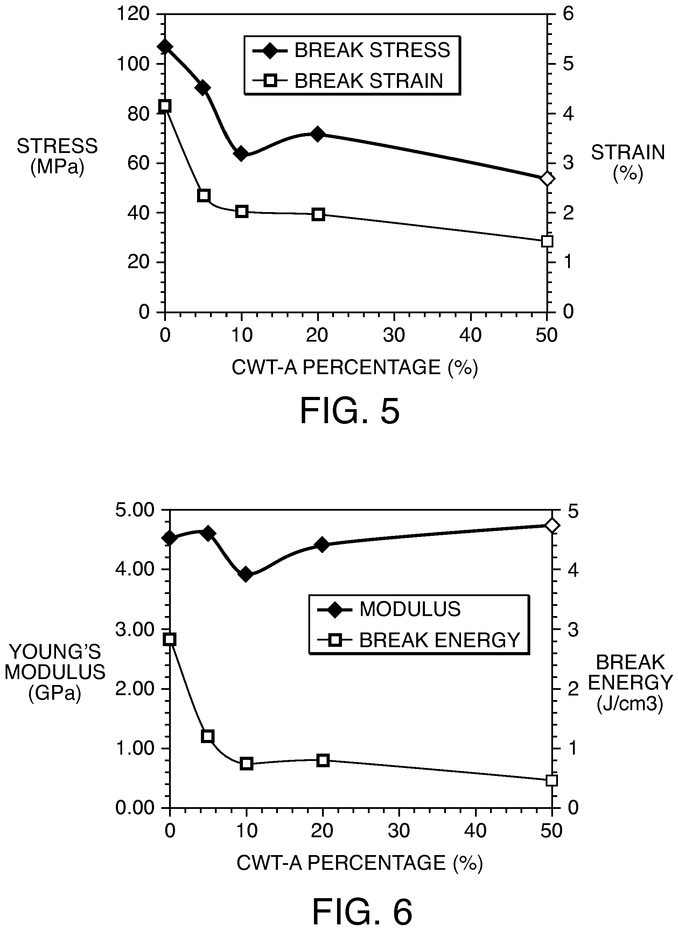

**FIGS. 5-10** show the test results from the tensile tests described above.

Referring to **FIG. 5**, a dual chart shows how the stress and strain vary with the percentage of antimicrobial in the tested film, the antimicrobial being zeolite. The greatest break stress occurs when the film contains 0% zeolite by weight. The break stress drops rapidly as zeolite is added, and plateaus somewhat at about 10% zeolite content by weight. Like the break stress, the break strain is greatest when the film contains 0% zeolite by weight, and generally plateaus after about 7% zeolite by weight has been added.

Referring to **FIG. 6****,** a dual chart shows how the elasticity and toughness vary with the percentage of antimicrobial in the tested film, the antimicrobial being zeolite. The greatest elasticity occurs when the film contains 50% zeolite by weight. The least amount of elasticity is seen at about 10% by weight zeolite. Like the break stress, toughness is greatest when the film contains 0% zeolite by weight, and generally plateaus after about 10% zeolite by weight has been added.

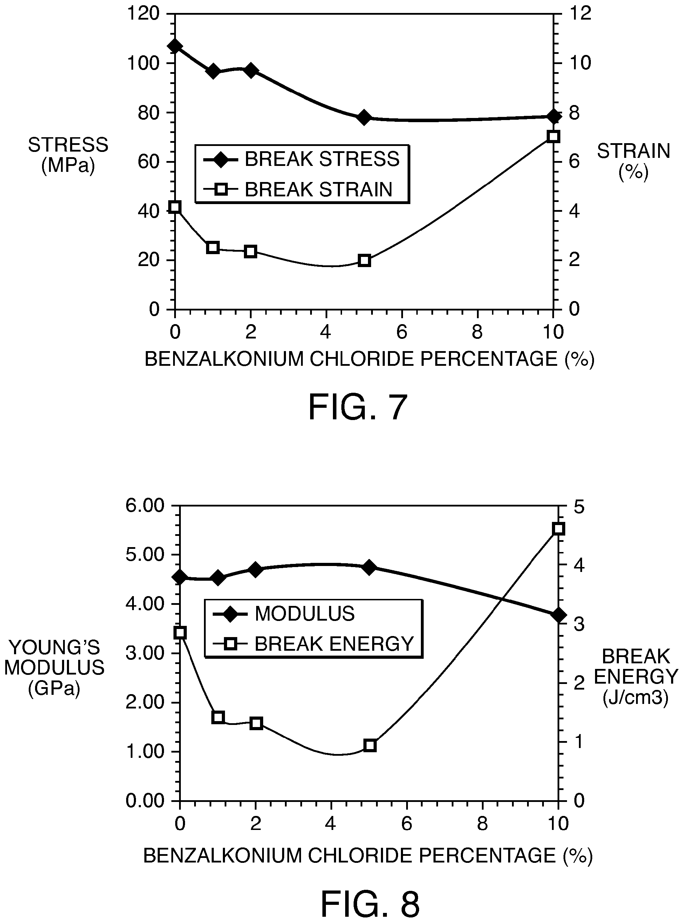

Referring to **FIG. 7****,** a dual chart shows how the stress and strain vary with the percentage of antimicrobial in the tested film, the antimicrobial being benzalkonium chloride. The greatest break stress occurs when the film contains 0% benzalkonium chloride by weight. The break stress drops as benzalkonium chloride is added, and plateaus somewhat at about 5% benzalkonium chloride content by weight. Unlike the break stress, the break strain is greatest when the film contains 10% benzalkonium chloride by weight, and generally plateaus when between about 1% and 5% benzalkonium chloride by weight has been added.

Referring to **FIG. 8****,** a dual chart shows how the elasticity and toughness vary with the percentage of antimicrobial in the tested film, the antimicrobial being benzalkonium chloride. The greatest elasticity occurs when the film contains 4% benzalkonium chloride by weight. The least amount of elasticity is seen at about 10% by weight benzalkonium chloride. Unlike the break stress, toughness is greatest when the film contains 10% benzalkonium chloride by weight has been added, a sharp rise from its lowest toughness that occurs at about 4% benzalkonium chloride by weight.

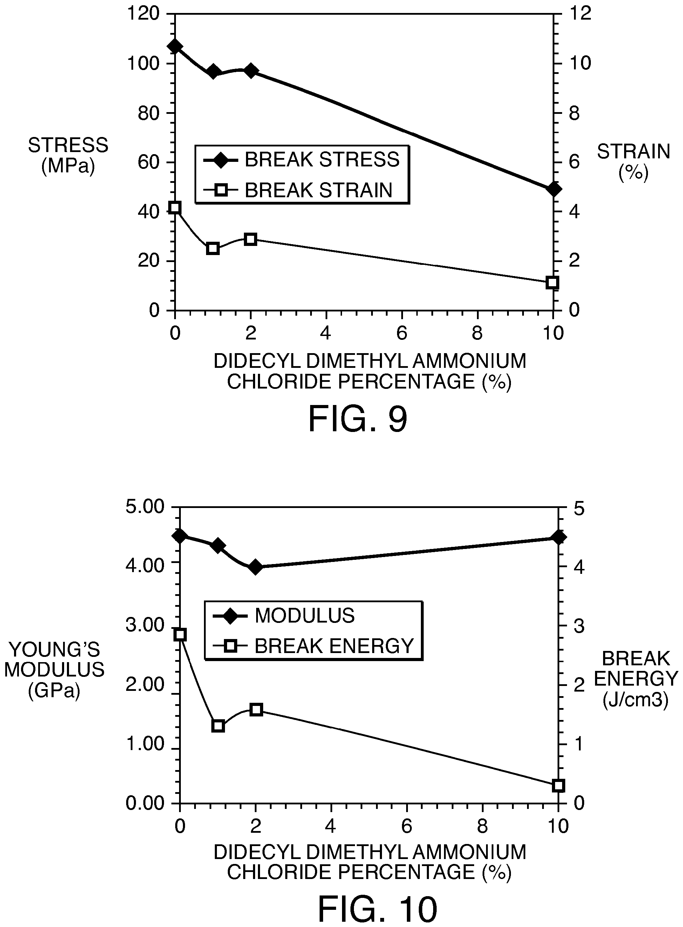

Referring to **FIG. 9****,** a dual chart shows how the stress and strain vary with the percentage of antimicrobial in the tested film, the antimicrobial being didecyl dimethyl ammonium chloride. The greatest break stress occurs when the film contains 0% didecyl dimethyl ammonium chloride by weight. The break stress drops as didecyl dimethyl ammonium chloride is added, with only a small plateau between about 1 to 2% didecyl dimethyl ammonium chloride by weight. Like the break stress, the break strain is greatest when the film contains 0% didecyl dimethyl ammonium chloride by weight, and drops somewhat steadily as it is added.

Referring to **FIG. 10**, a dual chart shows how the elasticity and toughness vary with the percentage of antimicrobial in the tested film, the antimicrobial being didecyl dimethyl ammonium chloride. The greatest elasticity occurs when the film contains either 0% or 10% didecyl dimethyl ammonium chloride by weight. The least amount of elasticity is seen at about 2% by weight didecyl dimethyl ammonium chloride. Like the break stress, toughness is greatest when the film contains 0% didecyl dimethyl ammonium chloride by weight has been added. TH toughness drops steadily after 2% didecyl dimethyl ammonium chloride by weight has been added to the film, following a minor plateau between the 1% and 2% didecyl dimethyl ammonium chloride by weight has been added to the film.

## Claims

1. A personal absorbent article selected from diapers, diaper pants, training pants, absorbent underpants, incontinence products, and urinary shields, the absorbent article comprising: an absorbent member disposed between a water-impermeable backsheet and a water-permeable liner, wherein the liner has a body-facing surface and an opposite garment-facing surface; and an extruded water-soluble article attached to the liner or to a surface of the absorbent member adjacent the liner, wherein the extruded water-soluble article comprises:
a homogeneous material comprising a water-soluble polymer having an extrusion temperature of 50 to 150°C; and
between 0.1% to 50% by weight of an active agent;
wherein the polymer is polyvinyl alcohol (PVOH); and
wherein the active agent is an antimicrobial.

2. The absorbent article of claim 1, wherein the polymer is an amorphous polyvinyl alcohol matrix having an extrusion temperature of 90°C to 125°C.

3. The absorbent article of claim 2, wherein there is 1% to 30% by weight of the active agent incorporated into the polyvinyl alcohol matrix.

4. The absorbent article of claim 1 wherein the extruded water-soluble article further comprises up to 50% starch by weight.

5. The absorbent article of claim 1 wherein the extruded water-soluble article further comprises up to 30% by weight of ethylene vinyl acetate copolymer.

6. The absorbent article of claim 1, wherein the extruded water-soluble article is a film.

7. The absorbent article of claim 6, wherein the film has a water dissolution speed from 5 seconds to 30 minutes as determined by a Water Dissolution Test of the present disclosure, or wherein the film has a basis weight of 5 g/m² to 500 g/m², or wherein the film has a tensile strength of 0.5 MPa to 50 MPa according to a Tensile Test of the present disclosure, or wherein the film has an elongation of 2% to 200% according to a Tensile Test of the present disclosure.

8. The absorbent article of claim 1 wherein the extruded water-soluble article further comprises a skin-benefit agent selected from the group consisting of prebiotics, probiotics, humidity control material, skin pH-control material, a skin protectant that mitigates skin irritation caused by feces/urine, and combinations thereof.

9. The absorbent article of claim 1 wherein the active agent is selected from the group consisting of didecyl dimethyl ammonium chloride, zeolite and benzalkonium chloride, and combinations thereof.

## Patentansprüche

1. Absorbierender Artikel für den persönlichen Gebrauch, der aus Windeln, Windelhosen, Trainingshosen, absorbierenden Unterhosen, Inkontinenzprodukten und Harnschutzeinlagen ausgewählt ist, der absorbierende Artikel Folgendes umfassend: ein absorbierendes Element, das zwischen einer wasserundurchlässigen Außenschicht und einer wasserdurchlässigen Auskleidung angeordnet ist, wobei die Auskleidung eine zum Körper weisende Fläche und eine gegenüberliegende, zur Kleidung weisende Fläche hat; und einen extrudierten, wasserlöslichen Artikel, der an der Auskleidung oder an einer Fläche des absorbierenden Elements neben der Auskleidung angebracht ist,
wobei der extrudierte, wasserlösliche Artikel Folgendes umfasst:
ein homogenes Material, das ein wasserlösliches Polymer mit einer Extrusionstemperatur von 50 °C bis 150 °C umfasst; und
zwischen 0,1 Gew.-% und 50 Gew.-% eines Wirkstoffs;
wobei das Polymer Polyvinylalkohol (PVOH) ist; und
wobei der Wirkstoff antimikrobiell ist.

2. Absorbierender Artikel nach Anspruch 1, wobei das Polymer eine amorphe Polyvinylalkoholmatrix mit einer Extrusionstemperatur von 90 °C bis 125 °C ist.

3. Absorbierender Artikel nach Anspruch 2, wobei 1 Gew.-% bis 30 Gew.-% des Wirkstoffs in der Polyvinylalkoholmatrix enthalten sind.

4. Absorbierender Artikel nach Anspruch 1, wobei der extrudierte, wasserlösliche Artikel ferner bis zu 50 Gew.-% Stärke umfasst.

5. Absorbierender Artikel nach Anspruch 1, wobei der extrudierte, wasserlösliche Artikel ferner bis zu 30 Gew.-% Ethylenvinylacetatpolymer umfasst.

6. Absorbierender Artikel nach Anspruch 1, wobei der extrudierte, wasserlösliche Artikel eine Folie ist.

7. Absorbierender Artikel nach Anspruch 6, wobei die Folie eine Wasserauflösungsgeschwindigkeit von 5 Sekunden bis 30 Minuten hat, wie durch einen Wasserauflösungstest der vorliegenden Offenbarung ermittelt, oder wobei die Folie ein Flächengewicht von 5 g/m² bis 500 g/m² hat, oder wobei die Folie eine Zugfestigkeit von 0,5 MPa bis 50 MPa gemäß einer Zugprüfung der vorliegenden Offenbarung hat oder wobei die Folie eine Verlängerung von 2 % bis 200 % gemäß einer Zugprüfung der vorliegenden Offenbarung hat.

8. Absorbierender Artikel nach Anspruch 1, wobei der extrudierte, wasserlösliche Artikel ferner ein Hautvorteilsmittel umfasst, das aus der Gruppe ausgewählt ist, die aus Präbiotika, Probiotika, Feuchtigkeitskontrollmaterial, Haut-pH-Kontrollmaterial, einem Hautschutzmittel, das Hautreizung lindert, die durch Fäkalien/Urin verursacht wird, und Kombinationen davon besteht.

9. Absorbierender Artikel nach Anspruch 1, wobei der Wirkstoff aus der Gruppe ausgewählt ist, die aus Didecyldimethylammoniumchlorid, Zeolit und Benzalkoniumchlorid und Kombinationen davon besteht.

## Revendications

1. Article absorbant à usage personnel choisi parmi des couches, des couches-culottes, des culottes d'apprentissages, des sous-vêtements absorbants, des produits d'incontinence et des pare-fuites urinaires, l'article absorbant comprenant : un élément absorbant disposé entre une feuille de renfort imperméable à l'eau et une doublure perméable à l'eau, dans lequel la doublure a une surface face au corps et une surface face au vêtement opposée ; et un article hydrosoluble extrudé attaché à la doublure ou à une surface de l'élément absorbant adjacente à la doublure,
dans lequel l'article hydrosoluble extrudé comprend :
un matériau homogène comprenant un polymère hydrosoluble ayant une température d'extrusion de 50 à 150 °C ; et
entre 0,1 % et 50 % en poids d'un agent actif ;
dans lequel le polymère est l'alcool polyvinylique (PVOH) ; et
dans lequel l'agent actif étant un antimicrobien.

2. Article absorbant selon la revendication 1, dans lequel le polymère est une matrice d'alcool polyvinylique amorphe ayant une température d'extrusion de 90 °C à 125 °C.

3. Article absorbant selon la revendication 2, dans lequel 1 % à 30 % en poids de l'agent actif sont incorporés dans la matrice d'alcool polyvinylique.

4. Article absorbant selon la revendication 1, dans lequel l'article hydrosoluble extrudé comprend en outre jusqu'à 50 % d'amidon en poids.

5. Article absorbant selon la revendication 1, dans lequel l'article hydrosoluble extrudé comprend en outre jusqu'à 30 % en poids de copolymère d'éthylène-acétate de vinyle.

6. Article absorbant selon la revendication 1, dans lequel l'article hydrosoluble extrudé est un film.

7. Article absorbant selon la revendication 6, dans lequel le film a une vitesse de dissolution dans l'eau de 5 secondes à 30 minutes, telle que déterminée par un test de dissolution dans l'eau de la présente divulgation, ou dans lequel le film a une masse surfacique de 5 g/m² à 500 g/m², ou dans lequel le film a une résistance à la traction de 0,5 MPa à 50 MPa selon un test de traction de la présente divulgation, ou dans lequel le film a un allongement de 2 % à 200 % selon un test de traction de la présente divulgation.

8. Article absorbant selon la revendication 1, dans lequel l'article hydrosoluble extrudé comprend en outre un agent bénéfique pour la peau choisi dans le groupe constitué par des prébiotiques, des probiotiques, une matière de régulation d'humidité, une matière de régulation du pH cutané, un agent de protection cutanée qui atténue l'irritation cutanée provoquée par les selles/urines, et des combinaisons de ceux-ci.

9. Article absorbant selon la revendication 1, dans lequel l'agent actif est choisi dans le groupe constitué par le chlorure de didécyl diméthyl ammonium, la zéolite et le chlorure de benzalkonium, et des combinaisons de ceux-ci.
